## Europäisches Patentamt
## European Patent Office
## Office européen des brevets

(11) Publication number: **0 078 154**
**A2**

(12) # EUROPEAN PATENT APPLICATION

(21) Application number: **82305599.1**

(22) Date of filing: **21.10.82**

(51) Int. Cl.³: **C 12 N 15/00**
C 12 P 21/00, C 12 N 1/00
C 02 F 3/34
//C12R1/19, C22B3/00

(30) Priority: **28.10.81 US 315808**

(43) Date of publication of application:
**04.05.83 Bulletin 83/18**

(84) Designated Contracting States:
**DE FR GB SE**

(71) Applicant: **THE REGENTS OF THE UNIVERSITY OF CALIFORNIA**
**2200 University Avenue**
**Berkeley, California 94720(US)**

(72) Inventor: **Baxter, John D.**
**131 San Pablo**
**San Francisco California(US)**

(72) Inventor: **Karin, Michael**
**1215 11th Avenue**
**San Francisco California(US)**

(74) Representative: **Paget, Hugh Charles Edward et al,**
**MEWBURN ELLIS & CO. 2/3 Cursitor Street**
**London EC4A 1BQ(GB)**

(54) **Plasmids and cells for heterologous expression of metallothioneins and processes for producing and using the metallothionein.**

(57) Methods and compositions are provided for producing metallothioneins by recombinant DNA techniques. An expression vector containing a heterologous gene coding for metallotioneins is produced and transformed into a suitable host organism, from which metallothionein can be expressed. The metallothioneins can be used *in vivo* or *in vitro* for extraction and concentration of heavy metals.

The invention described herein was made in the course of work under a grant or award from the Department of Health and Human Services.

EP 0 078 154 A2

# PLASMIDS AND CELLS FOR HETEROLOGOUS EXPRESSION OF METALLOTHIONEINS, AND PROCESSES FOR PRODUCING AND USING THE METALLOTHIONEIN.

Metallothioneins (MT) are a family of low molecular weight, heavy metal binding proteins. They are characterized by their high cystine content and lack of aromatic amino acids. The MT's bind 6-7 moles of heavy metals, such as zinc, cadmium, gold, silver, copper and mercury per mole of protein. They are ubiquitously distributed in the animal and plant kingdom, and are also found in prokaryotes.

In many situations it is desirable to be able to concentrate or segregate heavy metals. In the purification of water, mining, ore processing, or the like, it is desirable to have various means for removing heavy metal ions from solutions or dispersions of the metal ions.

The metallothioneins are naturally occurring compounds which provide an opportunity for selectively segregating a number of heavy metals. Furthermore, because their production is induced in vivo by the presence of heavy metals, microorganisms can be employed which will respond to increasing concentrations of the heavy metal by increasing the production of the metallothionein. Thus, one can have a naturally responsive system to variation in the concentration of heavy metals.

The regulation of metallothionein synthesis in vivo is described in Rudd and Herschman (1979) Tox. Appl. Pharmacol. 47:271276; Karin and Herschman (1979) Science 204:176-177; and Karin et al., (1980) Nature 286:295-297. Isolation of metallothionein is described in Karin and Herschman (1980) Eur. J. Biochem. 107:395-401. Descriptions of metallothionein sequences may be found in Kissling and Kagi (1977) Febs. Lett. 82:247-250; Huang et al., (1977) J. Biol. Chem. 252:8217-8221; and Glanville et al., (1981) Nature 292:267-269.

Methods are provided for the isolation and expression of genes coding for metallothioneins. The metallothioneins may then be used _in vitro_ and _in vivo_ for the isolation and concentration of heavy metals. The genes may be introduced into a wide variety of cells to enhance the production of the metallothioneins in the presence of heavy metals and to complement the endogenous production of metallothioneins.

The subject invention provides the various nucleotide sequences necessary for expression of metallothioneins. The joining of the DNA sequences to appropriate vectors for introduction of the metallothioneins into eukaryotic and prokaryotic cells, the expression of the metallothioneins in the cells and the use of the metallothioneins _in vivo_ and _in vitro_ for the concentration, segregation or isolation of heavy metal ions.

The metallothioneins are particularly effective in chelating metals of Groups IA and IIA of the periodic chart, particularly zinc, cadmium, mercury, copper, gold and silver. There are two major forms of the metallothioneins, which are distinguishable electrophoretically and chromatographically, known as metallothioneins 1 and 2. The two metallothioneins have a substantial degree of homology. The metallothioneins are relatively low molecular weight, approximately 6,000 daltons, with about 25 to 35 number percent of the amino acids being cystine.

The metallothioneins are present in a wide variety of species, including horse, human, rat, mouse, rabbit, chicken, mollusc, blue-green algae and yeast. In higher species they may be found in a number of different organs, such as liver, cervix, etc. These cells when employed for production of mRNA will conveniently be neoplastic, so as to be readily cultured _in vitro_.

The metallothionein genes may be obtained initially by a method which is generally applicable to cells which may be induced by an inducing agent, so as to substantially enhance the amount of messenger RNA which is transcribed from the gene of interest. A number of different reagents are known to induce the formation of metallothioneins. Included among these reagents are zinc, cadmium, glucocorticoid hormones, e.g. dexamethasome, which act as primary inducers, where the transcription may be further enhanced by the addition of secondary inducers, such as cycloheximide. See Karin et al., supra. By isolating the messenger RNA in accordance with conventional techniques, cDNA may then be produced employing reverse transcriptase. The resulting DNA may then be isolated and introduced into an appropriate vector to provide a plasmid for cloning in the vector. The plasmid may then be isolated, restricted to separate the sequences inserted into the vectors, and the inserted sequences separated from the vectors.

The total messenger RNA from induced cells and uninduced cells is then isolated and used to make labeled probes for hybridization, either RNA probes or DNA probes. Particularly, a radioactive label can be employed. The excision products from the plasmids are each individually combined with the probes from the induced cells and uninduced cells and the resulting hybridizations compared. Those excision products which show enhanced hybridization with the induced cells as compared with the uninduced cells are then isolated and selected for further consideration as the gene for the peptide of interest.

Desirably, there should be a distinguishing feature, for example a restriction site, which allows one a further means of selection for the peptide of interest. With the metallothioneins, a BamHI site is available near the N-terminus of the human $MT_{II}$ gene. The correctness of the determination may be further verified by restriction mapping, sequencing, providing for expression and detection of the metallothionein, or the like.

The gene may now be used for introduction into an appropriate vector which may then be introduced into a host cell recognized by the vector. Where cDNA has been employed, which will normally be the rule where the wild-type gene has introns, the cDNA will not have the promoter and terminator associated with the gene. Therefore, the gene will be introduced into a vector at an appropriate site providing for proper transcription of the metallothionein gene with the associated ribosomal start and stop sites. In certain situations, it may be desirable to determine the initiation codon, remove the 5'-flanking region and provide a different ribosomal start site, which may be more effective in the particular host.

One may choose a wide variety of vectors, depending upon the choice of host and the desired control signals, particularly for replication, such as copy number or the like. Various plasmids are available for $\underline{E}$. $\underline{coli}$ based on the F, R and ColI plasmids naturally present in $\underline{E}$. $\underline{coli}$. A number of plasmids are available for Gram-negative bacteria, based on the RK naturally occurring plasmids. For yeast, the 2μ-plasmid may be used or by employing a combination of the $\underline{ars}$ sequence with the centromere sequence, plasmids may be prepared which will be stably replicated in the host.

It is found among eukaryotes, that to varying degrees, different eukaryotes will recognize the replication regulatory signals from other hosts. By knowing the flanking and DNA sequences of the gene, one can determine the available restriction sites and a strategy developed for the insertion of the gene and appropriate regulatory signals into the replication systems containing vectors. Various techniques include finding an appropriate natually existing restriction site, which may be blunt ended or cohesive ended, making a blunt ended terminus from a cohesive ended terminus using $S_1$ nuclease, ligating short oligonucleotide linkers, so as to change the nature of the terminus and, as appropriate, introducing a regulatory sequence at an appropriate site. Numerous strategies have been described in the literature.

The plasmid will have in addition to the metallothionein gene with its associated regulatory signals and the replication system, other genes for a variety of purposes.

In some instances, it will be desirable to have more than one replication system, so that the plasmid may be cloned in a host different from the host in which the metallothionein is to be ultimately expressed. Thus, a plasmid may have both eukaryotic and prokaryotic replication systems. Other genes may provide means for selection, where one or more genes may be used to permit distinguishing between cells having the desired DNA sequence and lacking the desired DNA sequence. For example, by having a plasmid with two markers, one of which contains the restriction site for insertion of the metallothionein gene, one can distinguish between host cells having the plasmid containing the metallothionein gene from plasmids lacking the metallothionein gene. Markers include genes expressing proteins providing resistance to antibiotics, toxins, and heavy metals, as well as providing prototrophy to auxotrophic hosts. Other capabilities which may be built into the plasmid include temperature sensitivity, response to endogenous or exogenous chemicals, or the like.

When the desired plasmid has been completed, the plasmid may be introduced into the host cell in a variety of ways to provide a cell having an extrachromosomal functioning metallothionein gene providing expression of metallothionein. As already indicated, in some instances it may be desirable to initially clone the plasmid in a host different from the host in which expression of the metallothionein is desired. This can allow for increased amounts of the desired plasmid and greater purity. The plasmid may be introduced into the host cell in a variety of ways including transformation e.g. calcium shock, conjugation, transfection and transduction.

The host which is employed will be governed by a number of considerations, such as availability, ease of growth, ability to grow under the conditions of intended use, availability of appropriate vectors and regulatory signals, and the like. Many different hosts may be employed both prokaryotic and eukaryotic such as microorganisms e.g. bacteria, fungi e.g. yeast, protozoa, and algae. Methods

6

for introducing hybrid DNA into such microorganisms may be found in the literature. See, for example, Scherer and Davis (1979) DNAS USA 76:4951-4955; Struhl et al. (1979) ibid 76:1035-1039 and Stenchcomb et al. (1979) Nature 282:39-43. By appropriate choice of the host, the metallothionein may be employed in a variety of environments to concentrate heavy metals with the organism. In addition, by providing for leader sequences coding for secretion by the organism, it will be feasible to have the organisms secrete the metallothioneins into the environment to chelate the heavy metal ions.

Also, conveniently regulatory signals associated with a metallothionein gene may be joined to the metallothionein gene, whether from the same or different source, where such regulatory signals are recognized by the host. Thus, the production of metallothionein by expression of the extrachromosomal gene would be subject to the same or substantially the same regulation as the host chromosomal metallothionein gene. This would be particularly useful when induced overproduction of metallothionein is desired.

The plasmid may then be cloned and the plasmid isolated and the gene excised from the plasmid. Because of the homology between the metallothioneins from various sources, the gene may now be used as a probe for isolation from a variety of sources of either the DNA gene or the messenger RNA. In this manner, the genes expressing metallothioneins from a wide variety of eukaryotic and prokaryotic sources may be isolated, sequenced and by the procedures described above, used for expression of the particular metallothionein.

The resulting host having the enhanced capability of producing metallothionein may be used in a variety of ways. The host may be used as a source of metallothionein. The cell may be grown to provide the metallothionein production, followed by lysis and isolation from the lysate of the metallotionein. Isolation may be readily achieved, the metallothionein may be isolated and purified by ion-exchange chromatography and gel electrophoresis. See particularly
Karin and Herschman (1980), supra.

The isolated metallothionein may now be used to
chelate heavy metals, so as to reduce toxicity, or to maintain the heavy metals in solution, where it is desired to
keep the heavy metal solubilized. Thus the amount of the
free ions or metal salts may be diminished. Alternatively,
one may conjugate the metallothionein to a support, including particles, container surfaces, hollow fibers, and the
like. In this manner, the metallothionein may be used for
ion-exchange to remove heavy metals from various effluents,
such as chemical processing effluents, water treating plant
effluents, cooling tower effluents, ore processing effluents, and the like.

Instead of using the cell free metallothioneins,
one may use the cells themselves for sequestering the heavy
metals. Thus, by using techniques such as employed with
activated sludge, one can change the microflora in activated
sludge, to not only provide the degradation of organic
materials, but also to sequester heavy metals, where contamination by heavy metals is a matter of concern. In
addition, cells may be modified which are capable of growing
on heavy metal containing ores to permit concentration of
the metals in the cells, loosening of ore rock, or reducing
contamination of processing water containing heavy metals.

In order to demonstrate the subject invention, the
following examples are offered by way of illustration and
not by way of limitation.

EXPERIMENTAL

Total RNA was extracted by the method of Chirgwin
et al., (1979) Biochemistry 18:5294-5299, from HeLa cells
maximally induced to synthesize metallothionein by incuba-
tion in $10^{-5}$M $CdCl_2$ and $10^{-4}$M cycloheximide for 8hr. prior
to harvest, Poly(A)-containing RNA was selected by hybridization to oligo(dt) cellulose (Arviv and Leder (1972) PNAS
USA 72:3961-3965) and used as template for the synthesis of
double-stranded cDNA by sequential reverse transcriptase reactions. Following removal of hair-pin loops by $S_1$ nuclease digestion homopolymeric tracts (tails) with an average of 12 dCMP residues were added to the 3' termini of the cDNA by incubation with terminal transferase and dCTP (Chang et al., (1978), Nature 275:617-624). The double stranded, dCMP tailed cDNA sequences were annealed to plasmid pBR322 DNA, previously linearized with restriction endonuclease Pst I and 3'-tailed with an average of 10 dGMP residues. The resultant chimeric plasmid DNA was used to transform E. coli strain RRI. The yield of recombinant colonies was $4 \times 10^3$/µg of cDNA, greater than 90% being Amp$^S$, Tet$^r$ phenotype.

Bacterial colonies containing recombinant plasmids were grown and fixed on 0.45 micron nitrocellulose filters essentially as described by Grunstein and Hogness (1975) PNAS USA 72:3961-3965. Duplicate filters were hybridized with $^{32}$P-labelled cDNA synthesized from poly(A)-containing RNA from either (a) induced or (b) uninduced HeLa cells, as described above. Twenty-four colonies were judged by autoradiography to give a stronger hybridization signal with induced cDNA.

Plasmid DNA was prepared from each of these colonies and digested with BamHI and AvaII. Four colonies were found to contain a BamHI site within the cloned insert sequence and also to hybridize relatively strongly with a restriction fragment of a cloned mouse MT-I cDNA (Durnam et al., (1980) 77:6511-6515) containing only coding sequences. The identity of these sequences as coding for human metallothionein was verified by nucleic acid sequence analysis. Further restriction endonuclease analysis of these plasmids revealed the presence of a PvuII site within the cloned cDNA sequence. A BamHI-PvuII restriction fragment containing only human MT-II coding sequences from phMT$_{II}$-3 was nick-translated (Rigby et al., (1977) J. Mol. Biol. 113:237-251) and used as a probe to screen a third set of nitrocellulose filters containing the cDNA library for metallothionein cDNA clones. (c) cross-reacting sequences, presumed to be metallothioneins, were found at a frequency of about 2%.

In accordance with the subject invention, the gene DNA sequence is provided for the production of metallothioneins. The sequence can be used in isolating metallothionein genes from sources other than the human sequence. These genes are then introduced into appropriate vectors which may then be cloned for amplifying the amount of the gene. In addition, various host cells may be employed for introduction of the genes in a functional form, whereby metallothionein is over produced as compared to the base level of metallothionein produced by the host cell. In this manner, the host cells can be used as a source for metallothioneins which may then be isolated and employed in a variety of ways for the concentration, segregation or removal of heavy metals from a variety of sources, particularly aqueous effluents. In addition, various microflora may be modified to enhance their capability for segregating metals, particularly in the treatment of aqueous effluents, sewage, various processing fluids, and the like.

The human metallothionein mRNA and DNA (including cDNA) may be used diagnostically to determine genetic defects. By labelling the RNA or ssDNA, for example with a radionuclide such as $^{32}$P, and employing hybridization techniques and Southern blotting, the presence or absence of mutations in the host cells can be detected.

Although the foregoing invention has been described in some detail by way of illustration and example for purposes of clarity of understanding, it will be obvious that certain changes and modifications may be practiced.

The invention described herein was made in the course of work under a grant or award from the Department of Health and Human Services.

CLAIMS:

1. A host cell having an extrachromosomal functioning gene expressing a metallothionein.

2. A cell according to Claim 1, wherein said cell is a microorganism.

3. A cell according to Claim 2, wherein said microorganism is a bacterium.

4. A cell according to any of Claims 1, 2 or 3, wherein said expression is induced by the presence of heavy metals.

5. A cell according to Claim 1, wherein said gene is derived from cDNA.

6. A cell according to Claim 5, wherein said gene is a human gene.

7. A plasmid having a functional replication system for a microorganism host and a gene capable of expressing metallothionein.

8. A plasmid according to Claim 7, wherein said replication system is for a bacterium.

9. A plasmid according to any of Claims 7 or 8, wherein expression of said metallothionein gene is subject to heavy metal induction.

10. A plasmid according to Claim 7, wherein said metallothionein gene is derived from cDNA.

11. A plasmid according to Claim 10, wherein said metallothionein is human metallothionein.

12. A method for treating a heavy metal containing aqueous effluent which comprises:

combining said aqueous effluent with a microorganism containing an extrachromosomal gene capable of expressing metallothionein under conditions wherein said microorganism grows and produces metallothionein, whereby such heavy metals become chelated by said metallothionein.

13. A method according to Claim 12, wherein said metallothionein is secreted.

14. A method according to any of Claims 12 or 13, wherein said microorganism is a bacterium.

15. A method according to any of Claims 12 or 13, wherein said gene is derived from cDNA.

16. A method according to Claim 15, wherein said metallothionein is human metallothionein.

17. A method for producing metallothionein which comprises:

growing under appropriate nutrient conditions a host cell containing a functioning extrachromosomal gene expressing metallothionein, and isolating the metallothionein.

18. A method according to Claim 17, including the additional step of lysing said cells prior to isolation of said metallothionein.

19. A method according to Claim 17, wherein said metallothionein is secreted from said host cells.